# EUROPEAN PATENT APPLICATION

(11) **EP 2 039 777 A1**
(43) Date of publication of application: **25.03.2009**
(21) Application number: 07744851.2
(22) Date of filing: 07.06.2007
(51) Int. Cl.: C12P 19/26

(54) **METHOD FOR PURIFICATION OF HYALURONIC ACID SALT**

(30) Priority: 07.06.2006 JP 2006158155
(71) Applicant: Kyowa Hakko Bio Co., Ltd., Chiyoda-ku Tokyo 1008185 (JP)
(72) Inventor: MURATA, Hideki c/o Yamaguchi Production Center, 1-1, Kyowa-cho Hofu-shi, Yamaguchi 747/8522 (JP); FUKUDA, Kazuo c/o Logistics Center Kyowa Hakko Bio Co., Ltd., Chuo-ku, Tokyo 103-8503 (JP)
(74) Representative: Dunleavy, Kevin James
(86) International application number: PCT/JP2007/061521
(87) International publication number: WO 2007/142285

(57) **Abstract**

A partially purified product of a hyaluronic acid salt obtained from a culture of a microorganism capable of producing hyaluronic acid, preferably a microorganism belonging to the genus Streptcoccus, is brought into contact with a solution containing a salt and a hydrophilic organic solvent, thereby transferring to a liquid phase, impurities contained in said partially purified product such as proteins, nucleic acids, colorants, endotoxins and the like, and then isolating the hyaluronic acid salt as a precipitate.

## Description

### CROSS REFERENCE TO RELATED APPLICATIONS

This application claims priority under EPC, Article 87 based upon Japanese Patent Application Serial No. 2006-158155, filed on June 7, 2006. The entire disclosure of the aforesaid application is incorporated herein by reference.

### FIELD OF THE INVENTION

The present invention relates to a method for purification of hyaluronic acid salts.

### BACKGROUND OF THE INVENTION

Methods are known for producing hyaluronic acid salts including a method of extracting from living tissue containing hyaluronic acid, for example, cockscombs and purifying it, and a method of isolating from a culture medium obtained by culturing a microorganism capable of producing hyaluronic acid and purifying it. Since hyaluronic acid salts are polymers of molecular weights of several tens of thousands to several millions, this poses an important problem in purification in terms of the removal of polymeric impurities such as proteins, nucleic acids, pyrogens (endotoxin), and the like. This has resulted in known methods for preliminarily removing impurities before obtaining hyaluronic acid salts, such as a method of treating with an ion-exchange resin (See Patent reference 1, Japanese Examined Patent Application, Publication No. H5-56957); a method of treating with alumina (See Patent reference 2, Japanese Patent No. 2938880); a method of treating with silica gel (See Patent reference 3, Japanese Patent No. 2731546); a method of treating with a positively charged microfilter (See Patent reference 4, Japanese Unexamined Patent Application Publication No.H09-324001); a method of adding a quaternary ammonium salt (See Patent reference 5, Japanese Unexamined Patent Application Publication No. H11- 60608); and a method of pretreating disrupted products of cockscombs with a salt-containing organic solvent solution (See Patent reference 6, Japanese Unexamined Patent Application Publication No. S58-84801), and the like.

Since an aqueous solution of a hyaluronic acid salt is unstable, the final product is often obtained as a solid in industrial production thereof. Accordingly, hyaluronic acid salts are obtained as solids by adding a salt to an aqueous hyaluronic acid salt solution obtained by removing polymeric impurities as mentioned above, further adding a hydrophilic organic solvent, thereby depositing the hyaluronic acid salt as a precipitate, separating, and then drying it.

However, all the above-mentioned methods for the removal of polymeric impurities to obtain hyaluronic acid with high quality require a large volume of the solution to be used, complicating the process steps. Moreover, the materials used therein are expensive and the recovery rates of hyaluronic acid salts are low, resulting in high cost. Also known is a method for washing crude hyaluronic acid salt crystals derived from cockscombs with a salt-containing organic solvent solution, but it is not known that the endotoxin can be removed (See Patent reference 7, Japanese Unexamined Patent Application Publication No. S61- 171703).

It is an object of the present invention to produce hyaluronic acid salts with low impurity content at low cost in a simple operation.

### SUMMARY OF THE INVENTION

The present invention relates to the following (1) - (14).
(1) A method for purification of a hyaluronic acid salt comprising the steps of
   contacting a partially purified product of a hyaluronic acid salt obtained from a culture of a microorganism capable of producing hyaluronic acid with a solution containing a salt and a hydrophilic organic solvent to transfer impurities contained in said partially purified product into a liquid phase; and
   isolating the hyaluronic acid salt as a precipitate.
(2) The method for purification as set forth in the above (1), wherein the microorganism capable of producing hyaluronic acid is a microorganism belonging to the genus Streptococcus.
(3) The method for purification as set forth in the above (1) or (2), wherein the impurities contained in the partially purified product are those selected from the group consisting of proteins, nucleic acids, colorants, and endotoxins.
(4) The method for purification as set forth in the above (1) or (2), wherein the impurities contained in the partially purified product are endotoxins.
(5) The method for purification as set forth in any one of the above (1) to (4), wherein the hyaluronic acid salt is an alkaline metal or alkaline-earth metal salt.
(6) The method for purification as set forth in any one of the above (1) to (4), wherein the hyaluronic acid salt is a salt selected from the group consisting of a sodium salt, potassium salt, and calcium salt.
(7) The method for purification as set forth in any one of the above (1) to (4), wherein the hyaluronic acid salt is a sodium salt; and the salt contained in the solution is one or more types of salts selected from the group consisting of sodium chloride, sodium sulfate, and sodium acetate.
(8) The method for purification as set forth in any one of the above (1) to (4), wherein the hyaluronic acid salt is a sodium salt; and the salt contained in the solution is sodium chloride.
(9) The method for purification as set forth in any one of the above (1) to (4), wherein the hyaluronic acid salt is a potassium salt; and the salt contained in the solution is one or more types of salts selected from the group consisting of potassium chloride, potassium sulfate, and potassium acetate.
(10) The method for purification as set forth in any one of the above (1) to (4), wherein the hyaluronic acid salt is a calcium salt; and the salt contained in the solution is one or more types of salts selected from the group consisting of calcium chloride, calcium sulfate, and calcium acetate.
(11) The method for purification as set forth in any one of the above (1) to (10), wherein the hydrophilic organic solvent is a hydrophilic organic solvent selected from the group consisting of methanol, ethanol, propanol, isopropanol, and acetone.
(12) The method for purification as set forth in any one of the above (1) to (11), wherein the salt concentration of the solution is 3 to 6 % (w/v), and the methanol concentration is 55 to 65 % (v/v).
(13) The method for purification as set forth in any one of the above (1) to (11), wherein the salt concentration of the solution is 1 to 3 % (w/v), and the ethanol concentration is 44 to 60 % (v/v).
(14) The method for purification as set forth in any one of the above (1) to (11), wherein the salt concentration of the solution is 3 to 4 % (w/v), and the acetone concentration is 40 to 50 % (v/v).
   In accordance with the present invention, highly purified hyaluronic acid salts can be easily obtained at low cost, without using expensive materials or complicated treatment.

### DETAILED DESCRIPTION OF THE INVENTION

The hyaluronic acid salts which can be purified by the method of the present invention are preferably alkaline metal or alkaline earth metals salts of hyaluronic acid, more preferably, sodium, potassium, calcium salts thereof, or the like, and still more preferably a sodium salt thereof.

Although there are no particular limitations as to the average molecular weight of hyaluronic acid salts which can be purified by the method of the present invention; hyaluronic acid salts of any molecular weight can be purified, but hyaluronic acid salts with average molecular weights of 100,000 to 3 million are preferred.

Although there are no particular limitations as to the microorganisms capable of producing the hyaluronic acid as long as they are microorganisms with such capability, they are preferably those belonging to the genus Streptococcus, more preferably microorganisms of the species; Streptcoccus pyogenes, Streptcoccus equi, Streptcoccus equisimilis, Streptcoccus dysgalactiae, Streptcoccus zooepidemicus, or the like.

The microbial cultures include those that can be obtained by culturing said microorganism in media.

Any natural or synthetic culture medium can be used as long as it contains carbon sources, nitrogen sources, inorganic salts, and the like that can be assimilated by said microorganism and it allows an efficient culture of said microorganism.

The carbon sources may be any type that can be assimilated by microorganisms so that one can use carbohydrates such as glucose, fructose, sucrose, molasses which contain them, starch or starch hydrolysates, and the like; organic acids such as acetic acid , propionic acid , and the like; alcohols such as ethanol, and propanol, and the like.

The nitrogen sources include ammonia, inorganic or organic ammonium salts such as, ammonium chloride, ammonium sulfate, ammonium acetate, and ammonium phosphate, and the like; other nitrogen-containing compounds, and peptone, meat extract, yeast extract, corn steep liquor, casein hydrolysates, soybean cake and soybean cake hydrolysates, various fermentation microbial cells, the digested products thereof, and the like.

The inorganic salts that may be used include potassium dihydrogen phosphate, dipotassium hydrogen phosphate, magnesium phosphate, magnesium sulfate, sodium chloride, ferrous sulfate, manganese sulfate, copper sulfate, calcium carbonate, and the like.

Culture is performed under aerobic conditions, such as shake culture, aeration agitation culture, or the like. The recommended culture temperature is 15 to 50 °C, and the culture time is usually 16 hours to seven days. The pH during culture is preferably held at 3 to 9. The pH is adjusted with an inorganic or organic acid, alkaline solution, urea, calcium carbonate, ammonia, and the like.

Methods for obtaining a partially purified product of a hyaluronic acid salt from a microbial culture include a method of separating solids such as microbial cells and the like from a microbial culture by a solid-liquid separation such as filtration, centrifuge separation or the like , thereby obtaining a supernatant of the culture followed by adding a salt to said supernatant of the culture and then adding a hydrophilic organic solvent, whereby a hyaluronic acid salt is allowed to deposit and to stand or be centrifuged to precipitate the hyaluronic acid salt. Before or after the separation of solids such as microbial cells and the like by a solid liquid separation such as filtration, centrifugation or the like of a microbial culture, a base such as sodium hydroxide or an acid such as hydrochloric acid may be added to the microbial culture, thereby hydrolyzing the hyaluronic acid salts contained in the culture and depolymerizing the hyaluronic acid.

Any salt may be used in the above operation as long as it is sufficiently soluble in water, including, for example, sodium chloride, sodium sulfate, sodium acetate, potassium chloride, potassium sulfate, potassium acetate, and the like, preferably sodium chloride. The above salt may be used singly or as a combination thereof.

The hydrophilic organic solvents that may be used above include alcohols such as methanol, ethanol, propanol, and isopropanol, and ketones such as acetone.

The salt to be added to a supernatant of the culture is added so as to reach a preferred level of 1 to 30% (W/V). The concentration of the hydrophilic organic solvent added thereto thereafter may be at any level as long as the hyaluronic acid salt is practically insoluble at that concentration, but the addition is made to reach a preferred level of 30 to 70% (V/V).

Next, the hyaluronic acid salt precipitate is washed by contacting the partially purified product of the hyaluronic acid salt obtained above with a solution containing a salt and a hydrophilic organic solvent. Said solution may be added as is to the partially purified product obtained above. However, in order to improve the washing efficiency, one may remove most of the supernatant liquid from said partially purified product that consists of the hyaluronic acid salt and the supernatant liquid, obtain a mixture of a small amount of the supernatant liquid and the hyaluronic acid salt, and add said solution to said mixture. There are no limitations as to the amount of the supernatant liquid contained in said mixture, as long as, in the operation of removing impurities from the hyaluronic acid salt precipitate, the hyaluronic acid salt precipitate does not solidify in jellylike form nor is the purification efficiency much adversely affected; the amount thereof can be adjusted by those skilled in the art, in accordance with the types of the salt and hydrophilic organic solvent used for the purification. The amount of the supernatant liquid contained in said mixture is preferably, about 20% (v/v) or more, preferably about 20% or more but 100% (v/v) or less, more preferably, about 30% (v/v) but 80 % (v/v) or less, still more preferably about 50% (v/v), with respect to the hyaluronic acid salt precipitate in the mixture.

Methods for removing the supernatant liquids include those such as sedimentation, filtration, centrifugation, and the like.

Any salt may be acceptable for the solution containing a salt and a hydrophilic organic solvent to be used in the present invention as long as it is sufficiently soluble in water, including, for example, sodium chloride, sodium sulfate, sodium acetate, potassium chloride, potassium sulfate, potassium acetate, and the like, preferably sodium chloride. The above salts may be used singly or as a combination thereof.

The hydrophilic organic solvents contained in the solution used in the present invention include alcohols such as methanol, ethanol, propanol, and isopropanol, and ketones such as acetone.

Any concentrations of the salt and hydrophilic organic solvent are acceptable as long as the hyaluronic acid salt is practically insoluble and the impurities are soluble at those concentrations; for example, if methanol is used as a hydrophilic solvent, the salt concentration may be 3 to 5 % (w/v), preferably 5 % (w/v) and the methanol concentration may be 55 to 65 % (v/v) preferably, 55 to 60 % (v/v). If ethanol is used as a hydrophilic solvent, the salt concentration may be 1 to 3 % (w/v), preferably 2 % (w/v) and the ethanol concentration may be 44 to 60 % (v/v). If acetone is used as a hydrophilic solvent, the salt concentration may be 3 to 4 % (w/v) and the acetone concentration may be 40 to 50 % (v/v).

Contacting the partially purified product of a hyaluronic acid salt with the above-mentioned solution containing a salt and hydrophilic organic solvent allows the impurities contained in said partially purified product to be transferred to the liquid phase, whereby a mixture consisting of a hyaluronic acid salt precipitate and a supernatant liquid which contains impurities dissolved therein can be obtained; and a washed slurry of a hyaluronic acid salt freed of impurities can be obtained by removing the supernatant liquid from said mixture.

Impurities contained in the partially purified product of hyaluronic acid salts are proteins, nucleic acids, colorants, endotoxins and the like, preferably endotoxins derived from microorganisms.

The methods for removing the supernatant liquid containing impurities therein include separation on standing, filtration, centrifuge separation, and the like.

The above-mentioned operation which removes the impurities contained in the partially purified product of hyaluronic acid salts and obtains the washed slurry may be repeated to thoroughly remove impurities.

Next, the resultant washed slurry is contacted with a salt-free 70 to 100 % (v/v) hydrophilic organic solvent for washing the hyaluronic acid salt precipitate, followed by removing most of the supernatant liquid to thereby obtain a mixture consisting of a small amount of a supernatant liquid with an increased concentration of the hydrophilic organic solvent contained in the hyaluronic acid salt precipitate, and the hyaluronic acid salt precipitate. Said small amount of the supernatant liquid may preferably be about 20% (v/v) or more, preferably about 20% or more but 100% (v/v) or less, more preferably, 30% (v/v) or more but 80 % (v/v) or less, still more preferably about 50% (v/v), with respect to the amount of the hyaluronic acid salt precipitate.

The aforementioned washing operation using a highly concentrated, salt-free hydrophilic organic solvent may be repeated.

The aforementioned washing operation turns the hyaluronic acid salt precipitate from translucent gel form to white powder form; the separation and drying of said powdery substance enables one to obtain a dried product of high purity hyaluronic acid. The methods of separation include those such as filtration, centrifugation, and the like; the methods of drying include those such as vacuum drying and aeration drying, and the like.

The hydrophilic organic solvent can be recovered by distilling the supernatant liquid removed during the washing operation. The reuse of the recovered hydrophilic organic solvent allows reducing the volume of waste fluid and carrying out the present invention at low cost.

The present invention is described in detail with the following examples.

### Example 1

### Purification 1 using aqueous methanol solution

Streptococcus zooepidemicus NCTC7023 [J. Gen. Microbial.,15,485-491(1956)] was cultured at 37 °C for 16 hours in a Brain Heart Infusion agar culture medium (Manufactured by Nippon Seiyaku Co., Ltd) were inoculated into 300 mL of a seed medium composed of glucose 1 % (w/v), peptone (Manufactured by Kyokuto Seiyaku Co., Ltd) 1.5 % (w/v),yeast extract (Manufactured by Difco Co., Ltd) 0.5 % (w/v), corn steep liquor 1 % (w/v), sodium glutamate 0.3 % (w/v), dipotassium phosphate 0.2 % (w/v), magnesium sulfate 0.05 % (w/v), sodium thiosulfate 0.1 % (w/v), and calcium carbonate 2 % (w/v), followed by shake cultivation at 37 °C for 16 hours. The 150mL of a seed culture medium was inoculated into a 5 L volume jar fermentor holding 3 L of a fermentation culture medium (pH 7.2) composed of glucose 2.5 % (w/v), peptone 1.5% (w/v) ,yeast extract 0.5 % (w/v), corn steep liquor 0.5 % (w/v), dipotassium phosphate 0.2 % (w/v), magnesium sulfate 0.005 % (w/v), and sodium thiosulfate 0.1%(w/v), followed by cultivating at 37 °C, an aeration rate of 0.3 vvm, and pH 7.0 for 26 hours, whereby a culture medium containing hyaluronic acid salts with an average molecular weight of 2 million or more was obtained. Note that the molecular weight of the hyaluronic acid was determined using gel permeation chromatography (GPC).

To 400 mL of said culture was added sodium hydroxide so as to reach 0.5 mol/L, followed by stirring at 40 °C until the average molecular weight of the hyaluronic acid reached about 1 million or less. The resulting solution was adjusted to pH 7.0 with hydrochloric acid, and 26 g of activated carbon was added thereto, and the mixture was stirred at 40 °C for 1 hour. After stirring, it was filtered, and a partially purified filtrate was obtained.

To said partially purified filtrate was added sodium chloride so as to reach a final concentration of 5 % (w/v), followed by adding methanol so as to reach a final concentration of 55 % (v/v) and a partially purified deposited product of sodium hyaluronic acid (hereafter, a precipitate slurry) was obtained.

Said precipitate slurry was divided into two portions, each of which was centrifuged to remove most of the supernatant liquid to prepare a mixture consisting of about 50% (v/v) of a supernatant liquid with respect to the sodium hyaluronic acid precipitate and a sodium hyaluronic acid precipitate, and was submitted to the experiments below.
(1) The sodium hyaluronic acid precipitate was washed by stirring said mixture with about five times the volume in a volume ratio to said mixture of 90 % (v/v) aqueous methanol solution added thereto and then centrifuged, thereby sedimenting sodium hyaluronic acid. Most of the supernatant liquid was removed, whereby a mixture consisting of about 50% (v/v) of a supernatant liquid with respect to the sodium hyaluronic acid and a sodium hyaluronic acid precipitate was obtained.
After the same washing operation was repeated twice, the sodium hyaluronic acid precipitate was separated from the mixture and vacuum dried to obtain a dried product of sodium hyaluronic acid.
(2) The sodium hyaluronic acid precipitate was washed by stirring said mixture with about five times the volume in a volume ratio to said mixture of a 55 % (v/v) aqueous methanol solution containing 5%(w/v) sodium chloride added thereto, and then was centrifuged, thereby sedimenting sodium hyaluronic acid. Most of the supernatant liquid was removed to obtain a mixture consisting of about 50% (v/v) to the sodium hyaluronic acid of a supernatant liquid and a sodium hyaluronic acid precipitate.
The mixture obtained after the same washing operation was repeated twice was turned into a washed slurry and a 90% (v/v) aqueous methanol with about five times the volume in a volume ratio to the washed slurry was added thereto. The mixture was stirred, whereby the sodium hyaluronic acid precipitate was washed and then centrifuged, thereby sedimenting sodium hyaluronic acid. Most of the supernatant liquid was removed to obtain a mixture consisting of about 50% (v/v) of a supernatant liquid with respect to the sodium hyaluronic acid and a sodium hyaluronic acid precipitate.
After the same washing operation for the washed slurry was repeated twice, the sodium hyaluronic acid precipitate was separated from the mixture and vacuum dried to obtain a dried product of sodium hyaluronic acid.
The protein content of the obtained sodium hyaluronic acid was determined by the Lowry method. Results are shown in Table 1.

**[Table 1]**

| Washing operation with aqueous methanol solution containing sodium chloride | Protein Content (%) |
|---|---|
| (1) None | 0.36 |
| (2) Yes | 0.03 |

### EXAMPLE 2

### Purification 2 using aqueous methanol solution

290 mL of a culture containing a hyaluronic acid salt with an average molecular weight of 2 million or more was obtained by the same method as used in Example 1. To the culture was added sodium hydroxide so as to reach 0.4 mol/L, followed by stirring at 30 °C until the average molecular weight of the hyaluronic acid reached about 1 million or less. The resulting solution was adjusted to pH 7.0 with hydrochloric acid, and 13 g of activated carbon was added thereto, and the mixture was stirred at room temperature for 2 hours. After stirring, it was filtered, and a partially purified filtrate was obtained.

To said partially purified filtrate was added sodium chloride so as to reach a final concentration of 5 % (w/v), followed by adding methanol so as to reach a final concentration of 60 % (v/v), and a partially purified deposited product of sodium hyaluronic acid (hereafter, a precipitate slurry) was obtained.

Said precipitate slurry was divided into four portions, each of which was centrifuged to remove most of the supernatant liquid to prepare a mixture consisting of about 50% (v/v) of a supernatant liquid with respect to the sodium hyaluronic acid precipitate and sodium hyaluronic acid precipitate, and was submitted to the experiments below.
(1) The sodium hyaluronic acid precipitate was washed by stirring said mixture with about five times the volume in a volume ratio to said mixture of 100 % (v/v) methanol and then centrifuged, thereby sedimenting sodium hyaluronic acid. Most of the supernatant liquid was removed, whereby a mixture consisting of about 50% (v/v) of a supernatant liquid with respect to the sodium hyaluronic acid and a sodium hyaluronic acid precipitate was obtained.
   After the same washing operation was repeated twice, the sodium hyaluronic acid precipitate was separated from the mixture and vacuum dried to obtain a dried product of sodium hyaluronic acid.
(2) The sodium hyaluronic acid precipitate was washed by stirring said mixture with about five times the volume in a volume ratio to said mixture of a 55 % (v/v) aqueous methanol solution containing 5% (w/v) sodium chloride added thereto, and then centrifuged, thereby sedimenting sodium hyaluronic acid. Most of the supernatant liquid was removed to obtain a mixture consisting of about 50% (v/v) of a supernatant liquid with respect to the sodium hyaluronic acid, and a sodium hyaluronic acid precipitate.
   The mixture obtained after the same washing operation was repeated twice was turned into a washed slurry and stirred with about five times in a volume ratio to the washed slurry of 100 % (v/v) methanol added thereto, whereby the sodium hyaluronic acid precipitate was washed and then centrifuged, thereby sedimenting sodium hyaluronic acid. Most of the supernatant liquid was removed to obtain a mixture consisting of about 50% (v/v) of a supernatant liquid with respect to the sodium hyaluronic acid and sodium hyaluronic acid precipitate.
   After the same washing operation was repeated twice, the sodium hyaluronic acid precipitate was separated from the mixture and vacuum dried to obtain a dried product of sodium hyaluronic acid.
(3) The sodium hyaluronic acid precipitate was washed by stirring said mixture with about five times in a volume ratio to said mixture of a 60 % (v/v) aqueous methanol solution containing 5%(w/v) sodium chloride added thereto, and then centrifuged, thereby sedimenting sodium hyaluronic acid. Most of the supernatant liquid was removed to obtain a mixture consisting of about 50% (v/v) of a supernatant liquid with respect to the sodium hyaluronic acid and a sodium hyaluronic acid precipitate.
   The mixture obtained after the same washing operation was repeated twice was turned into a washed slurry and stirred with about five times in a volume ratio to the washed slurry of 100 % (v/v) methanol added thereto, whereby the sodium hyaluronic acid precipitate was washed and then centrifuged, thereby sedimenting sodium hyaluronic acid. Most of the supernatant liquid was removed to prepare a mixture consisting of about 50% (v/v) of a supernatant liquid with respect to the sodium hyaluronic acid and sodium hyaluronic acid precipitate.
   After the same washing operation for the washed slurry was repeated twice, the sodium hyaluronic acid precipitate was separated from the mixture and vacuum dried to obtain a dried product of sodium hyaluronic acid.
(4) The sodium hyaluronic acid precipitate was washed by stirring said mixture with about five times in a volume ratio to said mixture of a 65 % (v/v) aqueous methanol solution containing about 4% (w/v) sodium chloride added thereto, and then centrifuged, thereby sedimenting sodium hyaluronic acid. Most of the supernatant liquid was removed to obtain a mixture consisting of about 50% (v/v) of a supernatant liquid with respect to the sodium hyaluronic acid and a sodium hyaluronic acid precipitate.

The mixture obtained after the same washing operation was repeated twice was turned into washed slurry and stirred with about five times in a volume ratio to the washed slurry of 100 % (v/v) methanol added thereto, whereby the sodium hyaluronic acid precipitate was washed and then centrifuged, thereby sedimenting sodium hyaluronic acid. Most of the supernatant liquid was removed to obtain a mixture consisting of about 50% (v/v) of a supernatant liquid with respect to the sodium hyaluronic acid and a sodium hyaluronic acid precipitate.

After the same washing operation for the washed slurry was repeated twice, the sodium hyaluronic acid precipitate was separated from the mixture and vacuum dried to obtain a dried product of sodium hyaluronic acid.

The protein content of the resultant sodium hyaluronic acid was determined by the same method used in Example 1. Results are shown in Table 2.

**[Table 2]**

| Concentrations of sodium chloride and methanol in washing solution containing sodium chloride | Protein Content | |
|---|---|---|
| | (%) | |
| (1) Not washed with washing solution containing sodium chloride | 0.26 | |
| (2) Sodium Chloride 5%; Methanol 55% | 0.07 | |
| (3) Sodium Chloride 5%; Methanol 60% | 0.09 | 10 |
| (4) Sodium Chloride 4%; Methanol 65% | 0.12 | |

### EXAMPLE 3

### Purification 3 using aqueous methanol solution

6L (two batches from a 5L jar fermentor) of a culture medium containing hyaluronic acid salts with an average molecular weight of 2 million or more was obtained by the same method as used in Example 1. To the culture was added sodium hydroxide so as to reach 0.5 mol/L, followed by stirring at 30 °C until the average molecular weight of the hyaluronic acid reached about 1 million or less. The resulting solution was adjusted to pH 7.0 with hydrochloric acid, and 400 g of activated carbon was added thereto, and the mixture was stirred at 30 °C for 1.5 hours. After stirring, it was filtered, and a partially purified filtrate was obtained.

To said partially purified filtrate was added sodium chloride so as to reach a final concentration of 5 % (w/v), followed by adding methanol so as to reach a final concentration of 60 % (v/v), a partially purified deposited product of sodium hyaluronic acid (hereafter, a precipitate slurry) was obtained.

Said precipitate slurry was divided into two portions, which were left standing to sediment sodium hyaluronic acid, followed by removing most of the supernatant liquid from each portion to prepare a mixture consisting of about 50% (v/v) of a supernatant liquid with respect to the sodium hyaluronic acid precipitate and sodium hyaluronic acid precipitate, and was submitted to the experiments below.
(1) The sodium hyaluronic acid precipitate was washed by stirring said mixture with about 0.5 times the volume in a volume ratio to said mixture of 100 % (v/v) methanol added thereto and then was left standing, thereby sedimenting sodium hyaluronic acid. Most of the supernatant liquid was removed to obtain a mixture consisting of about 50% (v/v) of a supernatant liquid with respect to the sodium hyaluronic acid and a sodium hyaluronic acid precipitate.
   From the mixture obtained after the same washing operation was repeated 10 times, a sodium hyaluronic acid precipitate was separated and vacuum dried to obtain a dried product of sodium hyaluronic acid.
(2) The sodium hyaluronic acid precipitate was washed by stirring said mixture with about twice the volume in a volume ratio to said mixture of a 55 % (v/v) aqueous methanol solution containing 5%(w/v) sodium chloride added thereto, and then was left standing to sediment sodium hyaluronic acid. Most of the supernatant liquid was removed to prepare a mixture consisting of about 50% (v/v) of a supernatant liquid with respect to the sodium hyaluronic acid and a sodium hyaluronic acid precipitate.

The mixture obtained after the same washing operation was repeated three times was turned into a washed slurry and stirred with about 0.5 times the volume in a volume ratio to the washed slurry of 100 % (v/v) methanol added thereto, to wash the sodium hyaluronic acid precipitate, which was then left standing, thereby sedimenting sodium hyaluronic acid. Most of the supernatant liquid was removed to obtain a mixture consisting of about 50% (v/v) of a supernatant liquid with respect to the sodium hyaluronic acid.

From the mixture obtained after the same washing operation was repeated 10 times, a sodium hyaluronic acid precipitate was separated and vacuum dried to obtain a dried product of sodium hyaluronic acid.

Table 3 shows the pyrogen (endotoxin) content of the resultant sodium hyaluronic acid. The endotoxin content was determined using Toxicolor (Manufactured by Seikagaku Kogyo Co., Ltd.). Note that the endotoxin content was expressed in endotoxin units (EU) as defined by the general test method of the Japanese Pharmacopoeia (Endotoxin Assay method).

**[Table 3]**

| Concentrations of sodium chloride and methanol in washing solution containing sodium chloride | Pyrogen (endotoxin) content |
|---|---|
| | (EU/g) |
| (1) Not washed with washing solution containing sodium chloride | 96 |
| (2) Sodium Chloride 5%; Methanol 55% | 5 |

### EXAMPLE 4

### Purification 1 using aqueous ethanol solution

1.4 L of a culture obtaining hyaluronic acid salts with an average molecular weight of 2 million or more was obtained by the same method as used in Example 1. To the culture was added hydrochloric acid so as to bring the pH to 3.5, followed by stirring at 65 °C until the average molecular weight of the hyaluronic acid reached about 200,000. The resulting solution was adjusted to pH 7.0 with hydrochloric acid, and 15 g of activated carbon was added thereto, and the mixture was stirred at room temperature for 2 hours. After stirring, it was filtered, and a partially purified filtrate was obtained.

To said partially purified filtrate was added sodium chloride so as to reach a final concentration of 2 % (w/v), followed by adding ethanol so as to reach a final concentration of 60 % (v/v) and a partially purified deposited product of sodium hyaluronic acid (hereafter, a precipitate slurry) was obtained.

Said precipitate slurry was divided into two portions, which were left standing to sediment the deposited sodium hyaluronic acid, followed by removing most of the supernatant liquid of each portion to prepare a mixture consisting of about 50% (v/v) of a supernatant liquid with respect to the sodium hyaluronic acid precipitate and sodium hyaluronic acid precipitate, and were submitted to the experiments below.
(1) The sodium hyaluronic acid precipitate was washed by stirring said mixture with about 1.2 times the volume in a volume ratio to said mixture of an 85 % (v/v) ethanol solution added thereto, and then was left standing to sediment sodium hyaluronic acid. Most of the supernatant liquid was removed to obtain a mixture consisting of about 50% (v/v) of a supernatant liquid with respect to the sodium hyaluronic acid and a sodium hyaluronic acid precipitate.
   From the mixture obtained after the same washing operation was repeated 9 times, a sodium hyaluronic acid precipitate was separated and vacuum dried to obtain a dried product of sodium hyaluronic acid
(2) The sodium hyaluronic acid precipitate was washed by stirring said mixture with about three times the volume in a volume ratio to said mixture of a 60 % (v/v) aqueous ethanol solution containing 2 % (w/v) sodium chlorid, added thereto, and then was left standing, thereby sedimenting sodium hyaluronic acid. Most of the supernatant liquid was removed to prepare a mixture consisting of about 50% (v/v) of a supernatant liquid with respect to the sodium hyaluronic acid and a sodium hyaluronic acid precipitate.

The mixture obtained after the same washing operation was repeated three times was turned into a washed slurry and stirred with about 1.2 times the volume in a volume ratio to the washed slurry of 85 % (v/v) ethanol added thereto, to wash the sodium hyaluronic acid precipitate, which was then left standing, thereby sedimenting sodium hyaluronic acid. Most of the supernatant liquid was removed to obtain a mixture consisting of about 50% (v/v) of a supernatant liquid with respect to the sodium hyaluronic acid and a sodium hyaluronic acid precipitate.

After the same washing operation for the washed slurry was repeated 9 times, the sodium hyaluronic acid precipitate was separated from the resultant mixture and vacuum dried to obtain a dried product of sodium hyaluronic acid.

Table 4 shows the protein, nucleic acid, and colorant contents of the resultant sodium hyaluronic acid. The protein content was determined by the same method used in Example 1; the nucleic acid and colorant contents were determined against the OD260nm and OD430nm controls of a 0.3 (w/v) aqueous sodium hyaluronic acid solution, respectively.

**[Table 4]**

| Washing operation with aqueous | Protein | Nucleic Acid | Colorant Content |
|---|---|---|---|
| ethanol solution containing sodium | Content | Content | |
| chloride | (%) | (OD260nm) | (OD430nm) |
| (1) None | 0.62 | 0.264 | 0.004 |
| (2) Yes | 0.13 | 0.024 | 0.001 |

### EXAMPLE 5

### Purification 2 using aqueous ethanol solution

6L (two batches from a 5L jar fermentor) of a culture containing hyaluronic acid salts with an average molecular weight of 2 million or more was obtained by the same method as used in Example 1. To the culture was added sodium hydroxide so as to reach 0.4 mol/L, followed by stirring at 30 °C until the average molecular weight of the hyaluronic acid reached about 1 million or less. The resulting solution was adjusted to pH 7.0 with hydrochloric acid, and 400 g of activated carbon was added thereto, and the mixture was stirred at room temperature for 2 hours. After stirring, it was filtered, and a partially purified filtrate was obtained.

To said partially purified filtrate was added sodium chloride so as to reach a final concentration of 2 % (w/v), followed by adding ethanol so as to reach a final concentration of 44 % (v/v), and a partially purified deposited product of sodium hyaluronic acid (hereafter, a precipitate slurry) was obtained.

Said precipitate slurry was left standing to sediment the deposited sodium hyaluronic acid; and then most of the supernatant liquid was removed to obtain a mixture consisting of about 50% (v/v) of a supernatant liquid with respect to the sodium hyaluronic acid precipitate and a sodium hyaluronic acid precipitate.

The sodium hyaluronic acid precipitate was washed by stirring said mixture with about three times the volume in a volume ratio to said mixture of a 48 % (v/v) aqueous ethanol solution containing 2% (w/v) sodium chloride, added thereto, followed by standing to sediment the deposited sodium hyaluronic acid. Most of the supernatant liquid was removed to obtain a mixture consisting of about 50% (v/v) of a supernatant liquid with respect to the sodium hyaluronic acid and a sodium hyaluronic acid precipitate.

The mixture obtained after the same operation was repeated three times was turned into a washed slurry and said slurry was stirred with about 1.2 times the volume in a volume ratio to the washed slurry of 85 % (v/v) ethanol, added thereto, to wash the sodium hyaluronic acid precipitate, which was then left standing, thereby sedimenting sodium hyaluronic acid. Most of the supernatant liquid was removed to prepare a mixture consisting of about 50% (v/v) of a supernatant liquid with respect to the sodium hyaluronic acid and a sodium hyaluronic acid precipitate.

After the same washing operation for the washed slurry was repeated 9 times, the sodium hyaluronic acid precipitate was separated from said mixture and vacuum dried to obtain a dried product of sodium hyaluronic acid.

The protein and nucleic acid contents of the resultant sodium hyaluronic acid were determined by the same methods used in Example 4; the protein content was 0.07% and the OD260nm, an indicator of nucleic acid content, was 0.018.

### EXAMPLE 6

### Purification 1 using aqueous acetone solution

150 mL of a culture containing hyaluronic acid salts with an average molecular weight of 2 million or more was obtained by the same method as used in Example 1. To the culture was added sodium hydroxide so as to reach 0.4 mol/L, followed by stirring at 30 °C until the average molecular weight of the hyaluronic acid reached about 1 million or less. The resulting solution was adjusted to pH 7.0 with hydrochloric acid, and 7 g of activated carbon was added thereto, and the mixture was stirred at room temperature for 2 hours. After stirring, it was filtered, and a partially purified filtrate was obtained.

Said partially purified filtrate was divided into two portions and submitted to the experiments below.
(1) To the partially purified filtrate was added sodium chloride so as to reach a final concentration of 4 % (w/v), followed by adding acetone so as to reach a final concentration of 40 % (v/v), and depositing sodium hyaluronic acid to obtain a partially purified product of sodium hyaluronic acid (hereafter, a precipitate slurry). Said precipitate slurry was left standing to sediment the deposited sodium hyaluronic acid; and then most of the supernatant liquid was removed to obtain a mixture consisting of about 50% (v/v) of a supernatant liquid with respect to the sodium hyaluronic acid precipitate and a sodium hyaluronic acid precipitate.
   The sodium hyaluronic acid precipitate was washed by stirring said mixture with about three times the volume in a volume ratio to said mixture of a 40 % (v/v) aqueous acetone solution containing 4 %(w/v) of sodium chloride added thereto, and was left standing to sediment sodium hyaluronic acid. Most of the supernatant liquid was removed to obtain a mixture consisting of about 50% (v/v) of a supernatant liquid with respect to the sodium hyaluronic acid and a sodium hyaluronic acid precipitate.
   The mixture obtained after the same washing operation was repeated three times was turned into washed slurry and said slurry was stirred with about 1.2 times the volume in a volume ratio to said washed slurry of 80 % (v/v) acetone added thereto, to wash the sodium hyaluronic acid precipitate, which was then left standing, thereby sedimenting sodium hyaluronic acid. Most of the supernatant liquid was removed to obtain a mixture consisting of about 50% (v/v) of a supernatant liquid with respect to the sodium hyaluronic acid and a sodium hyaluronic acid precipitate.
   After the same washing operation for the washed slurry was repeated 9 times, the sodium hyaluronic acid precipitate was separated from the resultant mixture and vacuum dried to obtain a dried product of sodium hyaluronic acid.
(2) To said partially purified filtrate was added sodium chloride so as to reach a final concentration of 3 % (w/v), followed by adding acetone so as to reach a final concentration of 50 % (v/v), and depositing sodium hyaluronic acid to obtain a partially purified product of sodium hyaluronic acid (hereafter, a precipitate slurry). Said precipitate slurry was left standing to sediment the deposited sodium hyaluronic acid; and then most of the supernatant liquid was removed to obtain a mixture consisting of about 50% (v/v) of a supernatant liquid with respect to the sodium hyaluronic acid precipitate and a sodium hyaluronic acid precipitate.

The sodium hyaluronic acid precipitate was washed by stirring said mixture with about three times the volume in a volume ratio to said mixture of a 50 % (v/v) aqueous acetone solution containing 3 % (w/v) of sodium chloride added thereto, followed by standing to sediment sodium hyaluronic acid. Most of the supernatant liquid was removed to obtain a mixture consisting of about 50% (v/v) of a supernatant liquid with respect to the sodium hyaluronic acid and a sodium hyaluronic acid precipitate.

The mixture obtained after the same operation was repeated three times was turned into a washed slurry and said slurry was stirred with about 1.2 times the volume in a volume ratio to the washed slurry of 80 % (v/v) acetone added thereto, to wash the sodium hyaluronic acid precipitate, which was then left standing, thereby sedimenting sodium hyaluronic acid. Most of the supernatant liquid was removed to prepare a mixture consisting of about 50% (v/v) of a supernatant liquid with respect to the sodium hyaluronic acid and a sodium hyaluronic acid precipitate.

After the same washing operation for the washed slurry was repeated 9 times, the sodium hyaluronic acid precipitate was separated from the mixture and vacuum dried to obtain a dried product of sodium hyaluronic acid. The protein content of the resultant sodium hyaluronic acid was determined by the same method used in Example 1. Results are shown in Table 5.

**[Table 5]**

| Sodium chloride and acetone concentrations of washing solution containing | Protein |
|---|---|
| sodium chloride | content |
| | (%) |
| Sodium chloride 4%, Acetone 40% | 0.02 |
| Sodium chloride 3%, Acetone 50% | 0.03 |

### EXAMPLE 7

### Purification 2 using aqueous acetone solution

150 mL of a culture containing hyaluronic acid salts with an average molecular weight of 2 million or more was obtained by the same method as used in Example 1. To the culture was added sodium hydroxide so as to reach 0.4 mol/L, followed by stirring at 30 °C until the average molecular weight of the hyaluronic acid reached about 1 million or less. The resulting solution was adjusted to pH 7.0 with hydrochloric acid, and 7 g of activated carbon was added thereto, and the mixture was stirred at room temperature for 2 hours. After stirring, it was filtered, and a partially purified filtrate was obtained.

To the partially purified filtrate was added sodium chloride so as to reach a final concentration of 4 % (w/v), followed by adding acetone so as to reach a final concentration of 40 % (v/v), and a partially purified deposited product of sodium hyaluronic acid (hereafter, a precipitate slurry) was obtained.

Said precipitate slurry was divided into two portions, which were left standing to sediment the deposited sodium hyaluronic acid, followed by removing most of the supernatant liquid of each portion to prepare a mixture consisting of about 50% (v/v) of a supernatant liquid with respect to the sodium hyaluronic acid precipitate and sodium hyaluronic acid precipitate, and were submitted to the experiments below.
(1) The sodium hyaluronic acid precipitate was washed by stirring said mixture with about 1.2 times the volume in a volume ratio to said mixture of an 80 % (v/v) acetone solution added thereto, and then was left standing to sediment sodium hyaluronic acid. Most of the supernatant liquid was removed to obtain a mixture consisting of about 50% (v/v) of a supernatant liquid with respect to the sodium hyaluronic acid and a sodium hyaluronic acid precipitate.
After the same washing operation was repeated 9 times, the sodium hyaluronic acid precipitate was separated from the mixture and vacuum dried to obtain a dried product of sodium hyaluronic acid.
(2)The sodium hyaluronic acid precipitate was washed by stirring said mixture with about three times the volume in a volume ratio to said mixture of a 40 % (v/v) aqueous acetone solution containing 4 % (w/v) of sodium chloride added thereto, and then was left standing to sediment sodium hyaluronic acid. Most of the supernatant liquid was removed to obtain a mixture consisting of about 50% (v/v) of a supernatant liquid with respect to the sodium hyaluronic acid and a sodium hyaluronic acid precipitate.
The mixture obtained after the same washing operation was repeated three times was turned into washed slurry and said slurry was stirred with about 1.2 times the volume in a volume ratio to said washed slurry of 80 % (v/v) acetone added thereto, to wash the sodium hyaluronic acid precipitate, which was then left standing, thereby sedimenting sodium hyaluronic acid. Most of the supernatant liquid was removed to obtain a mixture consisting of about 50% (v/v) of a supernatant liquid with respect to the sodium hyaluronic acid and a sodium hyaluronic acid precipitate.
After the same washing operation for the washed slurry was repeated 9 times, the sodium hyaluronic acid precipitate was separated from the resultant mixture and vacuum dried to obtain a dried product of sodium hyaluronic acid.
Table 6 shows the pyrogen (endotoxin) content of the resultant sodium hyaluronic acid. The endotoxins content was determined by the same method as that of Example 3.

**[Table 6]**

| Sodium chloride and acetone concentrations of washing | Pyrogen (endotoxin) content |
|---|---|
| solution containing sodium chloride | (EU/g) |
| (1) Not washed with washing solution containing sodium | 137 |
| chloride | |
| (2) Sodium chloride 4%, acetone 40% | 2 |

### EXAMPLE 8

### Purification 3 using aqueous acetone solution

700 mL of a culture containing hyaluronic acid salts with an average molecular weight of 2 million or more was obtained by the same method as used in Example 1. To the culture was added sodium hydroxide so as to reach 0.4 mol/L, followed by stirring at 30 °C until the average molecular weight of the hyaluronic acid reached about 100,000. The resulting solution was adjusted to pH 7.0 with hydrochloric acid, and 20 g of activated carbon was added thereto, and the mixture was stirred at room temperature for 2 hours. After stirring, it was filtered, and a partially purified filtrate was obtained.

To the partially purified filtrate was added sodium chloride so as to reach a final concentration of 4 % (w/v), followed by adding acetone so as to reach a final concentration of 40 % (v/v), and a partially purified deposited product of sodium hyaluronic acid (hereafter, a precipitate slurry) were obtained.

Said precipitate slurry was allowed to stand whereby sodium hyaluronic acid was precipitated, followed by removing most of the supernatant liquid, and a mixture consisting of about 50% (v/v) of a supernatant liquid with respect to the sodium hyaluronic acid precipitate and sodium hyaluronic acid precipitate were obtained.

The sodium hyaluronic acid precipitate was washed by stirring said mixture with about three times the volume in a volume ratio to said mixture of an 40 % (v/v) acetone solution containing 4 %(w/v) of sodium chloride added thereto, and then was left standing to sediment sodium hyaluronic acid. Most of the supernatant liquid was removed to obtain a mixture consisting of about 50% (v/v) of a supernatant liquid with respect to the sodium hyaluronic acid and a sodium hyaluronic acid precipitate.

The mixture obtained after the same washing operation was repeated three times was turned into a washed slurry and said slurry was stirred with about 1.2 times the volume in a volume ratio to said washed slurry of 80 % (v/v) acetone added thereto, to wash the sodium hyaluronic acid precipitate, which was then left standing, thereby sedimenting sodium hyaluronic acid. Most of the supernatant liquid was removed to obtain a mixture consisting of about 50% (v/v) of a supernatant liquid with respect to the sodium hyaluronic acid and a sodium hyaluronic acid precipitate.

After the same washing operation for the washed slurry was repeated 9 times, the sodium hyaluronic acid precipitate was separated from the mixture and vacuum dried to obtain a dried product of sodium hyaluronic acid.

The protein content of the sodium hyaluronic acid was determined by the same method as that of Example 1 and was 0.03%.

### EXAMPLE 9

### Purification 4 using aqueous acetone solution

3.3L of a culture containing hyaluronic acid salts with an average molecular weight of 2 million or more was obtained by the same method used in Example 1. And the culture was diluted with water to 16.5 L, followed by being stirred at room temperate for 12 hours with 200 g of activated carbon added thereto. After stirring, it was filtered, and a partially purified filtrate was obtained.

To the partially purified filtrate was added sodium chloride so as to reach a final concentration of 4 % (w/v), followed by adding acetone so as to reach a final concentration of 40 % (v/v), and a partially purified deposited product of sodium hyaluronic acid (hereafter, a precipitate slurry) was obtained.

Said precipitate slurry was left standing to sediment the deposited sodium hyaluronic acid, followed by removing most of the supernatant liquid, and a mixture consisting of about 50% (v/v) of a supernatant liquid with respect to the sodium hyaluronic acid precipitate and sodium hyaluronic acid precipitate was obtained.

The sodium hyaluronic acid precipitate was washed by stirring said mixture with about three times the volume in a volume ratio to said mixture of an 40 % (v/v) acetone solution containing 4 %(w/v) of sodium chloride added thereto, and then was left standing to sediment sodium hyaluronic acid. Most of the supernatant liquid was removed to obtain a mixture consisting of about 50% (v/v) of a supernatant liquid with respect to the sodium hyaluronic acid and a sodium hyaluronic acid precipitate.

The mixture obtained after the same washing operation was repeated three times was turned into a washed slurry and said slurry was stirred with about 1.2 times the volume in a volume ratio to said washed slurry of 80 % (v/v) acetone added thereto, to wash the sodium hyaluronic acid precipitate, which was then left standing, thereby sedimenting sodium hyaluronic acid. Most of the supernatant liquid was removed to obtain a mixture consisting of about 50% (v/v) of a supernatant liquid with respect to the sodium hyaluronic acid and a sodium hyaluronic acid precipitate.

After the same washing operation for the washed slurry was repeated 9 times, the sodium hyaluronic acid precipitate was separated from the resulting mixture and vacuum dried to obtain a dried product of sodium hyaluronic acid.

The protein and nucleic acid contents of the resultant sodium hyaluronic acid were determined by the same methods used in Example 1 and Example 4, respectively; the protein content was 0.02% and the nucleic acid content was 0.006 (OD260nm).

### Potential Industrial Utility

According to the present invention, a method of producing hyaluronic acid salts with few impurities at low cost by a simple operation is provided.

## Claims

1. A method for purification of a hyaluronic acid salt **characterized by**,
contacting a resultant partially purified product of a hyaluronic acid salt obtained from a culture of a microorganism capable of producing hyaluronic acid with a solution containing a salt and a hydrophilic organic solvent to transfer impurities contained in said partially purified product into a liquid phase; and
isolating the hyaluronic acid salt as a precipitate from a mixture of said solution and said partially purified product of a hyaluronic acid salt.

2. The method for purification as set forth in Claim 1, wherein the microorganism capable of producing hyaluronic acid is a microorganism belonging to the genus Streptococcus.

3. The method for purification as set forth in Claim 1 or 2, wherein the impurities contained in the partially purified product are those selected from the group consisting of proteins, nucleic acids, colorants and endotoxins.

4. The method for purification as set forth in Claim 1 or 2, wherein the impurities contained in the partially purified product are endotoxins.

5. The method for purification as set forth in any one of Claims 1 to 4, wherein the hyaluronic acid salt is an alkaline metal or alkaline-earth metal salt.

6. The method for purification as set forth in any one of Claims 1 to 4, wherein the hyaluronic acid salt is a salt selected from the group consisting of a sodium salt, potassium salt and calcium salt.

7. The method for purification as set forth in any one of Claims 1 to 4, wherein the hyaluronic acid salt is a sodium salt; and the salts contained in the solution is one or more types of salts selected from the group consisting of sodium chloride, sodium sulfate and sodium acetate.

8. The method for purification as set forth in any one of Claims 1 to 4, wherein the hyaluronic acid salt is a sodium salt; and the salt contained in the solution is sodium chloride.

9. The method for purification as set forth in any one of Claims 1 to 4, wherein the hyaluronic acid salt is a potassium salt; and the salt contained in the solution is one or more types of salts selected from the group consisting of potassium chloride, potassium sulfate and potassium acetate.

10. The method for purification as set forth in any one of Claims 1 to 4, wherein the hyaluronic acid salt is a calcium salt; and the salt contained in the solution is one or more types of salts selected from the group consisting of calcium chloride, calcium sulfate and calcium acetate.

11. The method for purification as set forth in any one of Claims 1 to 10, wherein the hydrophilic organic solvent is a hydrophilic organic solvent selected from the group consisting of methanol, ethanol, propanol, isopropanol and acetone.

12. The method for purification as set forth in any one of Claims 1 to 11, wherein the salt concentration of the solution is 3 to 6 % (w/v), and the methanol concentration is 55 to 65 % (v/v).

13. The method for purification as set forth in any one of Claims 1 to 11, wherein the salt concentration of the solution is 1 to 3 % (w/v), and the ethanol concentration is 44 to 60 % (v/v).

14. The method for purification as set forth in any one of Claims 1 to 11, wherein the salt concentration of the solution is 3 to 4 % (w/v), and the acetone concentration is 40 to 50 % (v/v).
